# EUROPEAN PATENT SPECIFICATION

(11) **EP 1 384 726 B1**
(45) Date of publication and mention of the grant of the patent: **27.08.2008**
(21) Application number: 02759751.7
(22) Date of filing: 08.04.2002
(51) Int. Cl.: C07K 16/30, C07K 16/42, C12N 5/20, A61K 31/70, A61K 39/395

(54) **IMMUNOTHERAPEUTIC COMBINATIONS FOR THE TREATMENT OF TUMOURS THAT OVEREXPRESS GANGLIOSIDES**
IMMUNOTHERAPEUTISCHE KOMBINATIONEN ZUR BEHANDLUNG VON TUMOREN, DIE GANGLIOSIDE ÜBEREXPRIMIEREN
COMBINAISONS IMMUNOTHERAPEUTIQUES UTILISEES DANS LE TRAITEMENT DE TUMEURS SUREXPRIMANT LES GANGLIOSIDES

(30) Priority: 06.04.2001 CU 842001
(43) Date of publication of application: 28.01.2004
(73) Proprietor: Centro de Inmunologia Molecular, 12100 Ciudad Habana (CU); Fernández Molina, Luis Enrique, Ciudad de la Habana 12100 (CU); Vázquez López, Ana María, Ciudad de la Habana 10500 (CU); Pérez Rodríguez, Rolando, Ciudad de la Habana 10500 (CU); Pérez González, Alexis, Ciudad de la Habana 13100 (CU); Carr Perez, Adriana, Ciudad de la Habana 10500 (CU); Díaz Rodríguez, Yildian, Ciudad de la Habana 10300 (CU); Alfonso Fernández, Mauro, A, Ciudad de la Habana 10600 (CU)
(72) Inventor: FERNÁNDEZ MOLINA, Luis Enrique, Playa, Ciudad de la Habana 12100 (CU); VÁZQUEZ LÓPEZ, Ana Maria, Ciudad de la Habana 10500 (CU); PÉREZ RODRÍGUEZ, Rolando, Ciudad de la Habana 10500 (CU); PÉREZ GONZÁLEZ, Alexis, Ciudad de la Habana 13100 (CU); CARR PÉREZ, Adriana, Ciudad de la Habana 10500 (CU); DÍAZ RODRÍGUEZ, Yildian, Centro Habana, Ciudad de la Habana 10300 (CU); ALFONSO FERNÁNDEZ, Mauro A., Ciudad de la Habana 10600 (CU); ROJAS DEL CALVO, Adriana Maria, Centro Habana, Ciudad de la Habana 12400 (CU)
(74) Representative: Prins, Adrianus Willem
(86) International application number: PCT/CU2002/000002
(87) International publication number: WO 2002/081661

(56) References cited:
- EP-A- 0 586 002
- EP-A- 0 657 471
- EP-A- 0 661 061
- WO-A-99/20656
- US-A- 6 149 921

## Description

### Technical Field

This invention relates to human medicine, and especially to therapeutic vaccines that induce an immune response to tumors that over-express gangliosides.

### Prior Art

Gangliosides are components of the plasmatic membranes of the most of mammalian cells. Even when these glycosphingolipids are expressed in normal tissues, they are very attractive targets for immunotherapy due to the expression patterns during the malignant transformation of the cells (Hakomori, S. Ann. Rev. Biochem. 50; 1981:733-764; Hakomori, Cancer Res. 45; 1985:2405-2414; Irie, R. F., et al. In: Therapeutic monoclonal antibodies. Borrebaeck, C.A.K., and Larrick, J.W. (Eds.). M Stockton Press, 1990, pp. 75-94).

The saccharide nature of the gangliosides, together to the fact that they are self-antigens makes them very low immunogenic molecules. Some strategies have been used to increase the immunogenicity of these antigens. They are based on presentation of gangliosides to the immune system in a different molecular environment. One of these strategies has been the use of conjugated vaccines in which the saccharide antigen is covalently linked to a carrier protein where the carrier is very immunogenic for T cells. It makes possible to obtain a strong and long lasting antibody immune response. Using this procedure has been possible rise IgG antibodies against such gangliosides, although the titers after the re-immunizations were smaller in comparison with those obtained in a classic immune response against thymus-independent antigens (Helling, F et al. Cancer Res. 54; 1994:197-203; Helling, F et al. Cancer Res. 55; 1995:2783-2788; Livingston PO et al. Cancer Immunol Immunother 45; 1997:10-19).

New vaccine compositions have been reported to induce an immune response against N-acetylated and N-glycolylated gangliosides. These vaccines are based on hydrophobic conjugation between gangliosides and very small size proteoliposomes (VSSP) obtained from the association of the Outer Membrane Protein Complex (OMPC) from *Neisseria meningitidis, Gram-negative bacteria*, with gangliosides (gangliosides / VSSP) (Estévez F. Et al. Vaccine 18; 1999:190-197; Patents US 5,788,985 y US 6,149,921). The vaccines composed by GM3 ganglioside / VSSP or the (NeuGcGM3) ganglioside / VSSP have raised high IgM and IgG antibodies titers specific against GM3 and NeuGcGM3.

Otherwise, vaccination with GM3 / VSSP increased survival of mice bearing melanoma B16, it also reduced tumor volumes and it increased rejection rate to the subcutaneous transplant of the tumor (Alonso et al. Int. J Oncology 15; 1999: 59-66; Car, A. et al. Melanoma Research, in press).

The idiotype network theory proposed by Jeme 1974 (Jerne, N.K. Ann. Immunol. 125C; 1974:373-389), pointed out the immune system as an antibody network with a complex interaction pattern between them and several natural antigens, that interactions happen through the variable regions or idiotype (Id) and by interfacing idiotype-antiidiotype, the immune system is regulated. Jerne's theory supported new strategies for cancer active immunotherapy (AI) based on the use of anti-idiotype vaccines. The vaccination is carried out with an antibody (Ab1) that recognizes the tumor-associated antigen, which induces anti-idiotype antibodies (Ab2). These anti-idiotype antibodies mimic the nominal antigen and in turn they generate anti-anti-idiotype antibodies (Ab3) against the tumor-associated antigen (Schmolling J, et al. Hybridoma 14; 1995:183-186). On the other hand, the induction of immune response against tumor associated antigens has been reported after vaccination with anti-Id antibodies (Ab2) (Raychauhuri, S. et al. J. Immunol. 137; 1986:1743-1749; Raychauhuri, S. et al. J. Immunol. 139; 1987:3902-3910; Bhattacharya-Chatterjee, M. et al. J. Immunol. 139; 1987:1354-1360; Bhattacharya-Chatterjee, M. et al. J. Immunol. 141; 1988:1398-1403; Herlyn, D. et al. Intern. Rev. Immunol. 4; 1989:347-357; Chen, Z-J. et al. Cell. Imm. Immunother. Cancer, 1990:351-359; Herlyn, D. et al. In Vivo 5; 1991:615-624; Furuya, A. et al. Anticancer Res. 12; 1992:27-32; Mittelman, A. et al. Proc. Natl. Acad. Sci. USA 89; 1992:466-470; Durrant, LG. et al. Cancer Res. 54; 1994:4837-4840; Mittelman, A. et al. Cancer Res. 54; 1994:415-421; Schmitt, H. et al. Hybridoma 13; 1994:389-396; Chakrobarty, M. et al. J. Immunother. 18; 1995:95-103; Chakrobarty, M. et al. Cancer Res. 55; 1995:1525-1530; Foon, KA. et al. Clin. Cancer Res. 1; 1995:1285-1294; Herlyn, D. et al. Hybridoma 14; 1995:159-166; Sclebusch, H. et al. Hybridoma 14; 1995:167-174; Herlyn, D. et al. Cancer Immunol. Immunother. 43; 1996:65-76).

Another choice, concerning the use of Ab2 antibodies and in order to increase the immunogenicity of saccharide residues, involves the possibility that such saccharide epitopo can be represented by a protein epitopo on the antibody molecule. In fact, many of these anti-Id antibodies have been obtained; they mimic gangliosides highly expressed in tumor cells such as GM3, GD3 and GD2 (Yamamoto, S et al. J. Natl. Cancer Inst. 82; 1990:1757-1760; Chapman, P.B. et al. J. Clin. Invest 88; 1991:186-192; Cheung N-K V, et al. Int J Cancer 54; 1993:499-505; Saleh MN. Et al. J Immunol 151; 1993:3390-3398; Sen G. Et al. J Immunotherapy 21; 1998: 75-83).

Promissory results have been achieved from clinical trials in cancer patients by using Ab2 antibodies simultaneously with BCG or QS21 as adjuvant (McCaffery M. Et al. Clin Cancer Res 2; 1996: 679-686.; Foon KA. Et al. Clin Cancer Res. 4; 1998:1117-1124).

The monoclonal antibody P3 (Deposit Number ECACC 94113026) is known from the prior art, which specifically recognizes the sialic acid in N-glycolyl-containing monosialo and disialogangliosides. This Mab Ab1 recognizes antigens on human breast tumors and melanomas. (Patent US 5,788,985; Vázquez AM. Et al. Hybridoma 14; 1995:551-556; Moreno E. Et al. Glycobiology 8; 1998: 695-705; Marquina G. Et al. Cancer Res 56; 1996:5165-5171; Carr, A. Et al. Hybridoma 19 (3); 2000:241-247).

The anti-idiotype antibody 1 E10 (Ab2 MAb 1 E10) (Deposit Number ECACC 97112901) obtained by immunization with MAb P3 is a gamma type antibody (not internal image). Ab2 MAb 1E10 showed anti-tumor effect on growth tumors of breast and melanomas (Patent US 6,063,379; Vázquez et al. Hybridoma 14; 1995:183-186; Vázquez et al. Oncology Reports 7; 2000:751-756).

Do not exist evidences in the prior art about the use of the combination between vaccines comprising gangliosides and pharmaceutical compositions comprising anti-gangliosides antibodies (Ab1) or anti-idiotype antibodies (Ab2), named idiotypic vaccines; neither the use of combinations of idiotypic vaccine Ab1 with idiotypic vaccine Ab2.

The present invention refers the use of all the possible vaccine combinations described, in order to potentiate the effect that they produce each one of them separately.

### Detailed description of the invention

The present invention refers to an immunotherapeutic combination for the immunotherapy of tumors that over-express gangliosides, comprising a first vaccine and a second vaccine, wherein said first vaccine is a ganglioside vaccine comprising a ganglioside and said second vaccine is an idiotypic vaccine which comprises a murine monoclonal antibody and an adjuvant and is capable of eliciting an immune response against said monoclonal antibody, wherein said murine monoclonal antibody is either an Ab1 antibody against said ganglioside or an anti-idiotype Ab2 antibody against an antibody against said ganglioside.

Preferably that pharmaceutical composition, or combination of the pharmaceutical compositions, wherein said Ab1 antibody is the murine MAb P3 with Deposit Number ECACC 94113026; and where said Ab2 antibody is the murine anti-idiotype antibody 1E10 with Deposit Number ECACC 97112901 and where the vaccine comprises N-glycolyl GM3 (NeuGcGM3) or N-acetyl GM3 (NeuAcGM3) gangliosides.

In the present invention the first and second vaccine can be administered in a simultaneous or alternating way.

The compositions of this invention may be useful in the treatment of cancer, particularly those which over express gangliosides as well as lung, breast, digestive system, urogenital system, melanomas, sarcomas and those derived from neuroectodermic tissue.

The present invention also described the scheme for the administration of the pharmaceutical compositions, or combination of pharmaceutical compositions to treat mammalians.

In the present disclosure a method is also described that comprises the administration to mammals of the pharmaceutical composition, or combination of pharmaceutical compositions described previously for the prevention or treatment of tumors of breast, lung, digestive system, urogenital system, melanomas, sarcomas and of neuroectodermic origin.

### 1. Preparation of vaccine comprising gangliosides:

The vaccines comprising gangliosides are obtained according to the specification of Estevez and col in Vaccine 18, 1999: 190-197 and in Patents US 5,788,985 y US 6,149,921). Very Small Size Proteoliposomes (VSSP) obtained from the association of the Outer Membrane Protein Complex (OMPC) from the Gram-negative bacteria strain, *Neisseria meningitidis*, with synthetic or natural gangliosides incorporated therein (VSSP-G). The gangliosides could be (NeuGcGM3), GD3 or (NeuAcGM3).

### 2. Preparation of idiotypic vaccines:

The pharmaceutical compositions are obtained according to the specifications of Patents US 5,817,513 and US 6,063,379. The vaccine comprises murine anti-ganglioside monoclonal antibodies such anti-gangliósidos such as MAb P3 or murine anti-idiotype monoclonal antibodies such as Mab 1E10 that recognize anti-ganglioside monoclonal antibodies.

### 3. Immunotherapeutic combinations that potentiate the immune response and the antitumor effect of ganglioside vaccines and idiotypic vaccines in animal models:

The referred procedures can be used in mice or any other species of mammals. The components of the combination are the ganglioside vaccine and the idiotypic vaccine; they can be combined in different ways.

In one combination, the animals can be immunized with 3 to 10 doses in a range of 25 µg to 1 mg of the murine anti-ganglioside monoclonal antibody; the interval between doses can be 7 and 14 days. During this period the animals receive among 3 to 10 dose in a range among 60 to 1000 µg of the ganglioside vaccine, the interval between doses can be 7 to 14 days.

In another combination, the animals can be immunized with 3 to 10 dose in a range of 25 µg to 1 mg of the murine anti-idiotypic antibody specific against an anti-ganglioside antibody; the interval between doses can be 7 to 14 days. During this period the animals receive among 3 to 10 dose in a range among 60 to 1000 µg of the ganglioside vaccine, the interval between doses can be 7 to 14 days.

The administration of both types of vaccines can be simultaneous or alternating. The vaccines can be formulated as separate products or as a vaccine composition when they are administered in a simultaneous way. When vaccines are administered in an alternating way, the intervals between each type of vaccine can be 3-7 days. The vaccines are administered in an adjuvant that can be aluminum hydroxide (62.5 µg -2.5 mg), Montanide ISA 51 (0.1-1.2 ml/doses), or any other appropriate adjuvant. The total volume for each dose can be 10 µl - 2 ml. Vaccines comprising gangliosides can be administered intradermic, subcutaneous, intramuscular, intraperitoneal, intramucosal or their combinations. The same administration routes can be used for vaccines comprising antibodies.

The immunotherapeutic combinations increase the antibody immune response as well as the cellular immune response in treated animals.

The time of appearance of local tumors, the tumor volume and survival of treated subjects is compared with the same parameters for the control group, in order to evaluate the effectiveness of immunotherapeutic combinations. When comparing these parameters among treated and control groups it can be observed a shorter time of appearance of local tumors, a decrease of the tumor volume and an increase in survival for the group treated with therapeutic combinations of the present invention. The control groups are not only those animals treated with the adjuvant, but also those groups treated with just only one vaccine instead of with their combinations.

### 4. Immunotherapeutic combinations that potentiate the immune response and the antitumor effect of ganglioside vaccines and idiotypic vaccines in human patients:

The procedure before referred can also be applied to cancer patients in different clinical stages. Particularly those tumors that over express gangliosides as well as lung, breast, digestive system, urogenital system, melanomas, sarcomas and those derived from neuroectodermic tissue

In one combination, the patients can be immunized with 3 to 10 dose in a range of 0.1 to 5 mg of the murine anti-ganglioside monoclonal antibody; the interval between doses can be 7 and 14 days. During this period the patients receive among 3 to 10 dose in a range among 60 to 1000 µg of the ganglioside vaccine, the interval between doses can be 7 to 14 days.

In a second combination, the patients can be immunized with 3 to 10 dose in a range of 0.1 to 5 mg of the murine anti-idiotypic antibody specific against an anti-ganglioside antibody; the interval between doses can be 7 to 14 days. During this period the patients receive among 4 to 6 dose in a range among 60 to 1000 µg of the ganglioside vaccine, the interval between doses can be 7 to 14 days.

The administration of both types of vaccines can be simultaneous or alternating. The vaccines can be formulated as separate products or as a vaccine composition when they are administered in a simultaneously. When vaccines are administered in an alternating way, the intervals between each type of vaccine can be 3-7 days. The vaccines are administered in an adjuvant that can be aluminum hydroxide (1-5 mg/doses), Montanide ISA 51 (0.6-1.2 ml/doses), or any other appropriate adjuvant. The total volume for each dose can be 10 µl₋ 2 ml.

Vaccines comprising gangliosides can be administered intradermic, subcutaneous, intramuscular, intraperitoneal, intramucosal or their combinations. The same route can be used for vaccines comprising antibodies.

During vaccination, some biochemical parameters and antibodies titers are monitored by measurements in blood. The frequency can be 1 week to 3 months. Cellular immunity is studied using lymphocytes from the patients. The extractions are carried out with a frequency oscillating from one week to three months.

Finally, the patients are re-immunized with both vaccines at the concentrations before mentioned; the intervals between doses can be 1-6 months. They can be administered simultaneously or not and during 1 to 2 years.

The present vaccination scheme induces in patients an antibody and cellular immune response increased, and therefore to reduce tumor burden.

### EXAMPLES

### Example 1: Activation of anti-idiotype T2 and anti- anti-idiotype T3 cells in the syngeneic model, induced by immunization with murine MAB P3.

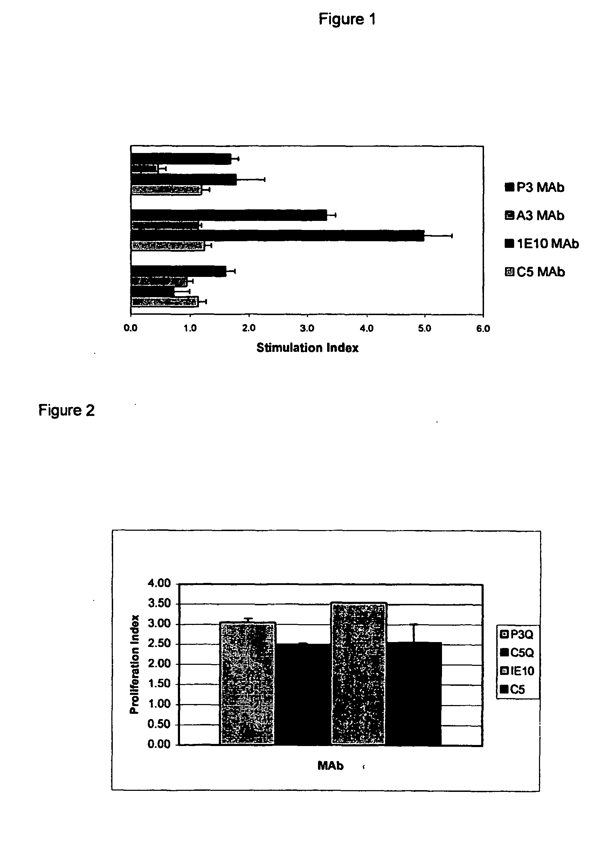

Female Balb/c and nude mice of the same genetic background 6-8 weeks old, were immunized subcutaneously with 100 µg of murine MAb P3 (anti-N-glycolylated ganglioside, Deposit Number ECACC 94113026; Vázquez et al., Hybridoma 14; 1995: 551-558; Moreno et al. Glycobiolgy 8; 1998: 695-705) and complete Freund's adjuvant (CFA). Seven days later mice were re-immunized with 50 µg of the antibody in incomplete Freund's adjuvant (IFA). At day 10, draining lymph nodes were collected and the lymphocytes were obtained by pressing with a syringe. The cellular suspension was used in experiments of cellular proliferation, which was measured by ³H-thymidine incorporation. In vitro lymphocyte proliferation was measured by cultivating lymphocyte suspension in the presence of increased concentrations (25 to 150 µg/mL) of the murine MAb P3 and its Ab2 MAb 1E10 (Number of Deposit ECACC 97112901). Isotype-matched murine MAbs were used as controls. Stimulation indexes equal to or higher than 3 where considered positive. Lymphocytes obtained after the immunization of Balb/c mice with P3 MAb specifically proliferated in the presence of this MAb and this proliferation was not observed when the cells were cultured in the presence of the murine control MAb A3 (IgM) (Alfonso M. and col.: Hybridoma 14; 1995: 209-216). This proliferation was dependent on the presence of T cells; because it was not obtained with lymph node cells from P3 immunized athymic nu/nu mice. Immunization with the murine MAb P3 not only induced the proliferation of T cells against the idiotype of this Ab1 MAb (T2), but also induced the proliferation of anti-anti-idiotype T cells (T3) specific for the murine Ab2 MAb 1E10 (IgG1), and not against Mab C5 of the same isotype (IgG1) (Figure 1).

### Example 2: Activation of anti-idiotype (T2) and anti-anti-idiotype (T3) induced by immunization with the chimeric antibody P3.

Chimeric antibody P3, whose amino acid sequences of its variable regions of the heavy (VH) and light (VL) chains are respectively shown in Figures 8 and 9, was used to immunize female Balb/c mice, 6-8 weeks old. For the first subcutaneous dose, 100 µg of the antibody emulsified in ACF was used. Seven days later animals were re-immunized with 50 µg of the antibody emulsified in AIF. At day 10, draining lymph nodes (LN) were collected and the LN cell lymphoproliferative capacity was analyzed by incubating in the presence of the murine and chimeric variants of antibodies P3 and Ab2 1E10, whose sequences of the variable regions are shown in the Figures 13 (heavy chain) and 14 (light chain). C5 murine MAb and its chimeric variant were used as isotype-matched controls (Patent Application WO 97/33916 A1). Lymphocytes from mice immunized with the chimeric antibody P3 specifically proliferated when cultivated in the presence of this antibody or the chimeric 1E10 MAb, and also when cultivated in the presence of the murine variants of these antibodies. Specificity of proliferation was demonstrated by using isotype-matched control MAbs. Therefore, chimeric P3 MAb maintains the ability of the murine variant to induce specific proliferation of anti-idiotype (T2) and anti-anti-idiotype (T3) T cells (Figure 2).

### Example 3: Reinforcement of the anti-tumor effect of the GM3-VSSP/Montanide ISA 51 vaccine by its combination with a vaccine composed by the murine MAb 1E10 adsorbed in aluminum hydroxide.

A group of 6-8 weeks old C57BL/6 female mice was immunized subcutaneously at days 0, 14, 28 and 42 with 50 µg of murine 1E10 MAb adsorbed in aluminum hydroxide. At days 7, 21, 35 and 49, mice received intramuscular doses with 120 µg of GM3-VSSP/Montanide ISA 51. Another group of mice was immunized in a similar way, but beginning the immunizations with the vaccine preparation containing GM3 ganglioside. Control groups separately immunized at the same time intervals and with the same number of doses of each vaccine or only with phosphate-buffered saline solution were used in these experiments. At day 63, mice in all groups were subcutaneously inoculated with 10 000 cells of the murine tumor B16. Tumor growth was evaluated in all groups.

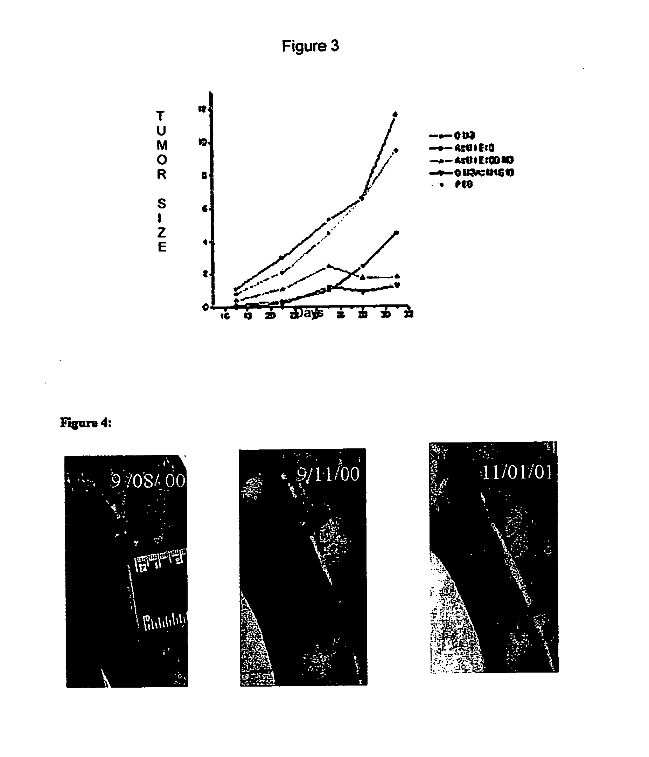

Mixed Lineal Pattern statistical data analysis was used for comparison of tumor growth between all groups and the group control that only received PBS. Vaccination with 1E10 MAb alone didn't protect mice against a challenge with 10,000 melanoma B16 cells. VSSP-GM3 vaccine caused a retard of the tumor growth (p <0.05). The combinations of GM3-VSSP and 1E10 MAb vaccines led to an increase in the protective effect observed with GM3-VSSP vaccine alone (p <0.05) (Figure 3).

### Example 4: Immunization schedule of cancer patients with NeuGc-GM3/VSSP ganglioside vaccine using Montanide ISA 51 as adjuvant.

Aiming to demonstrate the safety and immunogenicity of the immunization with the NeuGc-GM3 / VSSP vaccine using Montanide ISA 51 as adjuvant (Patent US 5,788,985 and US 6,149,921), a clinical trial was carried out in which 20 patients with advanced malignant melanoma, which were not eligible for any other onco-specific treatment, were immunized with the vaccine.

The patients received 9 doses of the vaccine preparation containing 200 µg of GM3 ganglioside. Doses were administered at days 0, 14, 28, 42, 56, 84, 112, 140, and 168. According to physician's criteria, patients received additional doses every 28 days after the sixth month until they completed one year of treatment.

Blood samples were obtained for routine biochemistry determinations and for evaluating serum titers of anti-NeuGcGM3 ganglioside antibodies at days 0, 14, 28, 56, 112, 168, 224, 280, and 332.

Antibodies titers were measured by ELISA. Serum dilutions were considered positive when anti-ganglioside OD values were equal or higher than 0,1 (referred to anti-methanol OD).

The toxicity of the vaccine NeuGcGM3-VSSP/Montanide ISA 51 consisted on erythema, local pain, induration in the place of injection and fever, and this allowed classification of the toxicity as mild, grade I/II according to the OMS criteria.

Patients developed specific antibody titers against NeuGcGM3 (between 1:80 and 1:2560). Detected antibody isotype included IgG and IgM (all patients) and IgG (75 % of the patients; Table 1).

In patient 01, which entered into the trial with a diagnosis of advanced malignant melanoma (Evolutionary Metastatic Disease, EMD), regression and stabilization of some cutaneous lesions was observed after two months of treatment, with uncolored halos around these lesions in which the presence of inflammatory infiltrate and necrosis was demonstrated by anatomopathologic studies in biopsies (Figure 4).

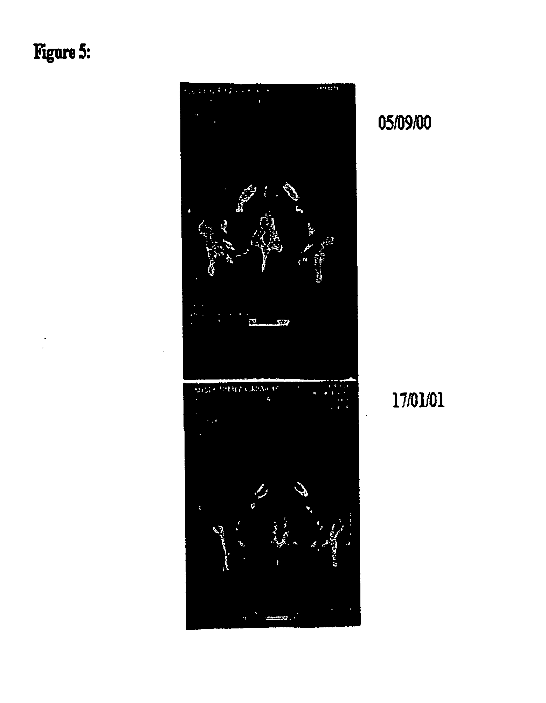

In patient 02, with diagnosis of advanced malignant melanoma, a stabilization of lung metastases lesion was observed in the right vertex 18 - 20 mm, during at least 4 months (Figure 5).

**TABLE I:**

| **Antibody titers against NeuGcGM3 in patients with advanced malignant melanoma immunized with the NeuGcGM3/ VSSP/ Montanide ISA 51 vaccine.** | | | | |
|---|---|---|---|---|
| **Patients** | **Day** | **IgM** | **IgG** | **IgA** |
| 01 | 0 | 0 | 0 | 0 |
| | 14 | 640 | 80 | 320 |
| | 28 | 320 | 160 | 320 |
| | 56 | 1280 | 160 | 320 |
| 02 | 0 | 160 | 160 | 160 |
| | 14 | 160 | 160 | 320 |
| | 28 | 160 | 160 | 320 |
| | 56 | 1280 | 320 | 320 |
| 03 | 0 | 80 | 320 | 0 |
| | 14 | 320 | 320 | 0 |
| | 28 | 640 | 320 | 0 |
| | 56 | 640 | 640 | 0 |
| 04 | 0 | 0 | 0 | 0 |
| | 14 | 160 | 80 | 160 |
| | 28 | 1280 | 80 | 160 |
| | 56 | 2560 | 640 | 2560 |

### Example 5: Immunization schedule in cancer patients with a idiotypic vaccine containing 1E10 Ab2 Mab Aluminum hydroxide-precipitated.

Aiming to demonstrate the safety and immunogenicity of an idiotype vaccine containing the murine anti-idiotype MAb 1E10 (Patent US 5,817,513 and US 6,063,379) and aluminum hydroxide as adjuvant, a clinical trial was carried out with 20 advanced malignant melanoma patients, which were not eligible for any other onco-specific treatment.

Patients received 4 doses of the vaccine, consisting in 2 mg of the 1E10 MAb. Blood samples were obtained before the treatment and 14 days after each immunization for routine biochemistry determinations and for evaluating serum titers of antibodies against 1E10 MAb idiotype and

NeuGc-GM3 ganglioside. Antibody titers were measured by an ELISA assay considering positive values those equal or higher than 0,15.

Vaccine administration to the patients produced mild toxicity, classified as degree I and II, according to the OMS.

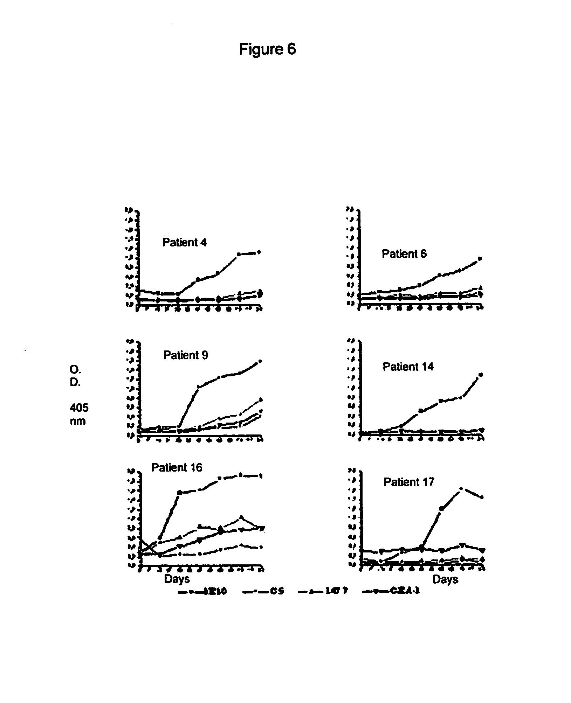

In 16 of the 17 valuable patients strong IgG Ab3 antibody responses were elicited, detectable after receiving 2 or 3 doses of the vaccine. The analysis of the specificity of this Ab3, showed a preferential recognition by patient's sera of 1E10 MAb, when compared with other isotype-matched control MAbs, which suggested the induction of idiotype-specific component in the antibody response to 1 E10 MAb. This was corroborated by the strong sera reactivity against the F(ab')2 fragments of this MAb, with a median titer of 1:15000 (titers from 1:10000 to more than 1:100000), with little or no recognition of the F(ab')2 fragments of the control MAbs used as controls.(Figure 6).

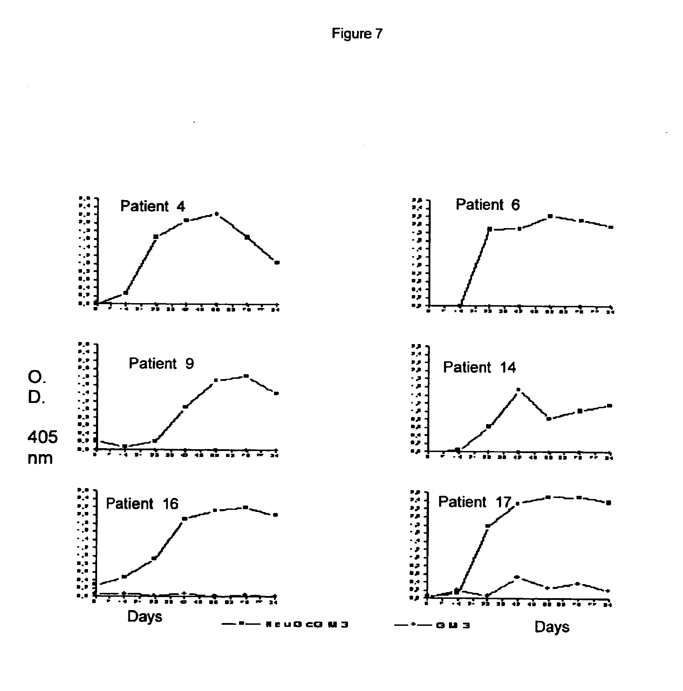

The antibodies generated against the NeuGcGM3 were, in most of the cases, both IgM and IgG, with titers 1:4000 and 1:800, respectively (Figure 7).

A patient with diagnosis of malignant melanoma and liver metastases was included in the clinical trial; after the treatment it was shown stabilization of the disease for 8 months and the patient's survival was 15 months.

### Example 6: Combined immunization with anti-idiotypic vaccine comprising Ab2 MAb1E10 and NeuGcGM3-VSSP ganglioside vaccine.

A melanoma patient with metastases, which had been submitted to monthly surgery, after the diagnosis, received 6 doses of the idiotypic vaccine containing Ab2 MAb1E10 and aluminum hydroxide gel (2 mg of the MAb per dose), days 0,14,28,42,56. During this period, also it was administered to the patient the ganglioside vaccine containing 200ug NeuGcGM3-VSSP ganglioside and Montanide ISA 51 as adjuvant, days 7, 21, 35, 49, 63. After the patient received this immunization scheme, he was re-immunized monthly with both vaccines simultaneously during two month. For the period of vaccination new lesions didn't appear and the patient condition was good.

### Example 7: Recognition of tumor tissues by the MAb 14F7.

NeuGcGM3 ganglioside is recognized by MAb 14F7 (patent application WO 99/40119). Recognition of tumor tissues by the antibody is shown.

Formalin fixed tissue was included in paraffin. The histology was evaluated in hematoxillin-eosin colored sections.

These sections were immune staining with the complex biotin-estreptavidin- peroxidase (Hsu, S. M. y Raine, L. 1981,J. Histochem Cytochem., 29:1349-1353). Briefly, the paraffin was removed and wet sections were treated with H₂O₂ 3% (in methanol solution) for 30 minutes, to reduce endogenous peroxidase activity. After incubation with MAb 14F7 (pure) for 1 hour at room temperature, biotinylated anti-mouse immunoglobulins and streptavidin-peroxidase complex were added (Dakopatts) at room temperature; between incubation times, the sections were washed with a Tris-HCl buffer solution. The reaction (POD) it was developed with 5 mL of Tris-HCl, 0,005 mL buffer solution containing H₂O₂ to 30% and of 3-3 diaminobencidine 3 mg.

After washing with hematoxillin-contrasted water (Mayer), the sections were covered with balm and covered. The enzyme reaction renders a brown color.

The fresh biopsies from pathological tissues were obtained one hour after surgery. All the tissues were washed with saline solution, and frozen in liquid nitrogen immediately and they were conserved frozen at -80° C.

The frozen fragments were cut in a cryostat Leica at -25°C. Serial sections with 5 um were obtained, air dried and used immediately or conserved at -20°C enveloped in aluminum paper; afterward the sections were fixed with para-formaldehyde 4% for 20 minutes.

In Table II it is shown the immunostaining with MAb 14F7 in several human tumors. A strong membrane and cytoplasm staining is appreciated in more than 50% of tumor cells. The dye was very intense in colon carcinomas, uterus, ovary, sarcomas, lymph nodes and brain metastases of breast cancer, as well as melanoma metastases.

**TABLE II:**

| **Tumor lmmunostaining with MAb 14F7.** | |
|---|---|
| **Tumor** | **Immunostaining (Positives/Total)** |
| Colon Carcinomas | 9/9* |
| Uterus Carcinomas | 2/2* |
| Ovary Carcinomas | 2/2* |
| Sarcomas | 2/2* |
| Breast Tumors and lymph node metastases | 6/6* |
| Melanoma metastases | 2/2* |

| | |
|---|---|
| * More than 50% of cells showed membrane and cytoplasm intensely immunostained. | |

### Brief description of the figures:

**Figure 1****.** Mice Balb/c euthymic and athymic mice were immunized subcutaneously with 100 µg of the murine MAb P3 in CFA followed by a re-immunization with 50 µg of the MAb emulsified in IFA. After three days, lymph nodes cells were collected and it was carried out the proliferation assay with different concentrations of the MAbs P3, A3, 1 E10 and C5.
**Figure 2****.** Mice Balb/c were immunized subcutaneously with 100 µg of the chimeric antibody P3 in CFA followed by a re-immunization with 50 µg of the antibody emulsified in IFA. After three days, lymph nodes cells were collected and it was carried out the proliferation assay with different concentrations using murine and chimeric antibodies as control.
**Figure 3****.** Kinetics of growth for murine B16 tumor in mice immunized with the immunotherapeutic combinations, specifically the idiotypic vaccine comprising the Ab2 MAb1E10 and of the GM3-VSSP ganglioside vaccine, as it is detailed in the Example 3.
**Figure 4****:** Evolution of skin metastases in melanoma patient. The pictures were taken previous to the immunization with the vaccine NeuGcGM3 / VSSP / ISA 51, 2 and 4 months after the treatment. Uncolored halos around some lesions are observed, stabilized lesions, in one lesion a diminished size was observed and in one lesion increment was detected.
**Figure 5****:** Evolution of lung metastases in a melanoma patient. The lung right vertex observed by computer assisted axial tomography, the lesion size was 18x20 mm before the immunization with the vaccine NeuGcGM3 / VSSP / ISA 51 and 4 months after the immunization stabilization of the lesion is observed.
**Figure 6****:** Recognition of F(ab')2 fragments from Ab2 MAb1E10 and from other MAbs with the same isotype by serum from patients immunized with the idiotypic vaccine containing Ab2 MAb1E10 and aluminum hydroxide gel as adjuvant.
**Figure 7****:** Recognition of GM3(NeuGc) and GM3(NeuAc) gangliosides serum from patients immunized with the idiotypic vaccine containing Ab2 MAb1E10 and aluminum hydroxide gel as adjuvant.

## Claims

1. An immunotherapeutic combination for the immunotherapy of tumors that over-express gangliosides, comprising a first vaccine and a second vaccine, wherein said first vaccine is a ganglioside vaccine comprising a ganglioside and said second vaccine is an idiotypic vaccine which comprises a murine monoclonal antibody and an adjuvant and is capable of eliciting an immune response against said monoclonal antibody, wherein said murine monoclonal antibody is either an Ab1 antibody against said ganglioside or an anti-idiotype Ab2 antibody against an antibody against said ganglioside.

2. Immunotherapeutic combination according to claim 1, wherein said Ab1 antibody is anti-ganglioside MAb P3 produced by the hybridoma identified by the Deposit Number ECACC 94113026.

3. Immunotherapeutic combination according to claim 1 ; wherein said Ab2 antibody is MAb 1E10 produced by the hybridoma identified by the Deposit Number ECACC 97112901.

4. Immunotherapeutic combination according to any one of claims 1-3, wherein said ganglioside is the NeuGcGM3 ganglioside.

5. Immunotherapeutic combination according to any one of claims 1-3, wherein said ganglioside is the NeuAcGM3 ganglioside.

6. Immunotherapeutic combination according to any one of claims 1-5, wherein the administration of said vaccines is simultaneous or alternating.

7. Use of an immunotherapeutic combination according to any one of claims 1-6 in the preparation of an agent for the treatment of tumors that over-express gangliosides.

8. The use according to claim 7 in the preparation of an agent for the treatment of tumor of breast, lung, digestive system, urogenital system, melanomas, sarcomas and those from neuroectodermic tissues.

## Patentansprüche

1. Inimuntherapeutische Kombination für die Immuntherapie von Tumoren, die Ganglioside überexprimieren, umfassend eine erste Vakzin und eine zweite Vakzin, wobei die erste Vakzine eine Gangliosid-Vakzine ist, die ein Gangliosid umfasst, und die zweite Vakzin eine idiotypische Vakzin ist, die einen murinen monoklonalen Antikörper und ein Adjuvans umfasst und in der Lage ist, eine Immunantwort gegen den monoklonalen Antikörper zu erzeugen, wobei der murine monoklonale Antikörper entweder ein Ab1-Antikörper gegen das Gangliosid oder ein anti-idiotypischer Ab2-Antikörper gegen einen Antikörper gegen das Gangliosid ist.

2. Immuntherapeutische Kombination gemäß Anspruch 1, bei der der Ab1-Antikörper Anti-Gangliosid Mab P3 ist, der von dem Hybridom produziert wird, welches durch die Hinterlegungsnummer ECACC 94113026 identifiziert wird.

3. Immuntherapeutische Kombination nach Anspruch 1, bei der der Ab2-Antikörper Mab 1E10 ist, der von dem Hybridom produziert wird, welches durch die Hinterlegungsnummer ECACC 97112901 identifiziert wird.

4. Immuntherapeutische Kombination nach einem der Ansprüche 1 bis 3, bei der das Gangliosid das NeuGcGM3-Gangliosid ist.

5. Immuntherapeutische Kombination nach einem der Ansprüche 7, bis 3, bei der das Gangliosid das NeuAcGM3-Gangliosid ist.

6. Immuntherapeutische Kombination nach einem der Ansprüche 1 bis 5, bei der die Verabreichung der Vakzine gleichzeitig oder nacheinander erfolgt.

7. Verwendung einer immuntherapeutischen Kombination nach einem der Ansprüche 1 bis 6 bei der Herstellung eines Mittels zur Behandlung von Tumoren, die Ganglioside überexprimieren.

8. Verwendung nach Anspruch 7 bei der Herstellung eines Mittels für die Behandlung von Tumoren von Brust, Lunge, Verdauungssystem, Urogenitialsystem, Melanomen, Sarkomen oder solchen von neuroektodermen Geweben.

## Revendications

1. Combinaison immunothérapeutique pour l'immunothérapie de tumeurs qui surexpriment des gangliosides, comprenant un premier vaccin et un deuxième vaccin, dans laquelle ledit premier vaccin est un vaccin gangliosidique qui comprend un ganglioside et ledit deuxiè me vac cin est un vaccin idiotypique qui comprend un anticorps monoclonal murin et un adjuvant et qui est capable de déclencher une réponse immunitaire contre ledit anticorps monoclonal, ledit anticorps monoclonal murin étant soit un anticorps Ab1 dirigé contre ledit ganglioside, soit un anticorps anti-idiotype Ab2 dirigé contre un anticorps dirigé contre ledit ganglioside.

2. Combinaison immunothérapeutique selon la revendication 1, dans laquelle ledit anticorps Ab 1 est un anticorps monoclonal anti-ganglioside P3 produit par l'hybridome identifié par le numéro de dépôt ECACC 94113026.

3. Combinaison immunothérapeutique selon la revendication 1, dans laquelle ledit anticorps Ab2 est l'anticorps monoclonal 1E10 produit par l'hybridome identifié par le numéro de dépôt ECACC 97112901.

4. Combinaison immunothérapeutique selon l'une quelconque des revendications 1 à 3, dans laquelle ledit ganglioside est le ganglioside NeuGcGM3.

5. Combinaison immunothérapeutique selon l'une quelconque des revendications 1 à 3, dans laquelle ledit ganglioside est le ganglioside NeuAcGM3.

6. Combinaison immunothérapeutique selon l'une quelconque des revendications 1 à 5, dans laquelle l'administration desdits vaccins est simultanée ou alternée.

7. Utilisation d'une combinaison immunothérapeutique selon l'une quelconque des revendications 1 à 6 pour préparer un agent destiné à traiter des tumeurs qui surexpriment des gangliosides.

8. Utilisation selon la revendication 7 pour préparer un agent destiné à traiter des tumeurs du sein, du poumon, du système digestif, du système urogénital, des mélanomes, des sarcomes et des tumeurs de tissus neurectodermiques.
